# EUROPEAN PATENT APPLICATION

(11) **EP 3 260 143 A1**
(43) Date of publication of application: **27.12.2017**
(21) Application number: 16752641.7
(22) Date of filing: 12.02.2016
(51) Int. Cl.: A61L 27/04

(54) **BIODEGRADABLE MAGNESIUM AND METHOD FOR CONTROLLING DEGRADATION RATE OF BIODEGRADABLE MAGNESIUM**

(30) Priority: 17.02.2015 KR 20150023925
(71) Applicant: SNU R&DB Foundation, Seoul 08826 (KR)
(72) Inventor: SHIN, Kwang-seon, Seoul 06601 (KR)
(74) Representative: Krauns, Christian
(86) International application number: PCT/KR2016/001425
(87) International publication number: WO 2016/133311

(57) **Abstract**

The present invention relates to biodegradable magnesium and a method for preparing the same. According to the present invention, biodegradable magnesium is characterized in that its biodegradation rate is controlled using the difference in atomic packing density.

## Description

### [Technical Field]

The present invention relates to biodegradable magnesium and a method for controlling the degradation rate particularly in magnesium in a living body by using crystallographic anisotropy according to the crystal orientation of a single crystal magnesium or polycrystalline magnesium with textured structure oriented in a specific crystallographic orientation, and magnesium with an in vivo degradation rate controlled by the method.

### [Background]

Technological advancements in modern medicine have allowed implants to be used for surgical purposes, such as the attachment or fixation of skin tissues or bones. Since implants that remain in the body after treatment may induce various complications, the implants must be onerously removed through additional procedures after achieving their purpose.

Meanwhile, stents are placed in clogged arteries to expand blood vessels and allow blood to flow more freely. When the stent has been in place for a long time, its metal surface may induce coronary artery thrombosis and lead to the sudden death of the patient who has undergone the procedure.

For these reasons, many attempts have been made to make the materials of implants and stents biodegradable, leading to proposals to use biodegradable materials such as polymeric materials, ceramic-based materials and metal-based materials.

Polymeric materials such as polylactic acid (PLA) and polyglycolic acid (PGA), and copolymers such as poly(lactic-co-glycolic acid)(PLGA) have low mechanical strength, and are not suitable to be used in the orthopedic field or as dental implants.

Additionally, ceramic materials such as tricalcium phosphate (TCP) have a low impact resistance due to inherent brittleness. Therefore, ceramic materials are not safe to use as biomaterials as they easily fracture.

Accordingly, the demand to develop biodegradable metals has been great as they have the potential to possess great strength, workability and ductility. Metals such as magnesium, iron and tungsten have been proposed as prospective biodegradable metals. Magnesium, in particular, is attracting attention as the most suitable biodegradable material. Magnesium alloys have started being used as parts of fixing screws for bonding bones.

The degradation rate of the biodegradable material in living bodies should proceed in proportion to the regeneration rate of a tissue. If the degradation rate is too fast and stability is lost before the damaged tissue recovers, the medical device will not perform its required functions. If the degradation rate is too slow, it could cause the aforementioned problems.

Therefore, controlling the degradation rate of biodegradable magnesium is a factor that must be considered in the design of biodegradable magnesium-based medical devices.

Magnesium has advantages in that it has sufficient strength and is malleable. Weaknesses of magnesium include that it is low in in vivo corrosion resistance and dissolves too quickly, which is the main factor preventing the application of magnesium to various medical devices.

As a method for controlling the degradation rate of magnesium, Korean Patent Laid-Open Publication No. 2010-0053480 discloses the method of improving strength and corrosion resistance by adding alloy elements such as Zr, Mo, Nb, Ta, Ti, Sr, Cr, Mn, Zn, Si, P, NI and Fe to Mg. Since biodegradable magnesium has to be applied to the living body, components harmful to the living body cannot be used as alloy elements, and thus, there is a limit to the improvement of corrosion resistance and control of the degradation rate through alloying.

Also, Korean Patent Laid-Open Publication No. 2010-0123428 reveals a method for controlling the degradation rate of magnesium in vivo through a difference in the increasing rate of the pH of electrolytes in vivo and a difference in the amount of generated hydroxyl ions resulting from a degradation reaction of magnesium in vivo by controlling the surface area of magnesium exposed to a living body.

Using the method of Korean Patent Laid-Open Publication No. 2010-0123428, it is possible to control the degradation rate by controlling the surface area of a given magnesium material. However, the method of Korean Patent Laid-Open Publication No. 2010-0123428 is not to control the intrinsic degradation rate of the magnesium material in contact with the living body. It is difficult to apply the method to a case where the degradation rate is lowered to a certain level because the degradation rate is too fast compared to the in vivo restoration rate of a tissue.

### [Disclosure]

### [Technical Problem]

The present invention provides magnesium with a degradation rate which is determined by controlling the crystallographic anisotropy of a surface in contact with a living body.

The present invention also provides a method for determining the in vivo degradation rate of magnesium by controlling the crystallographic anisotropy of a surface in contact with a living body.

The present invention further provides a medical article employing magnesium having a surface contacting a living body, the degradation rate of the contact surface of the magnesium being determined by controlling the crystallographic anisotropy.

### [Technical Solution]

According to the first aspect of the present invention, the provided biodegradable magnesium has a biodegradation rate which is determined by controlling an atomic packing density of a surface in contact with a living body.

In the first aspect, magnesium may include pure magnesium or a magnesium alloy.

In the first aspect, the magnesium may be a single crystal magnesium and the surface of the magnesium may be oriented with a specific crystal plane.

In the first aspect, the magnesium may be polycrystalline magnesium, comprised of a texture which is preferentially oriented with a specific crystal plane.

In the first aspect, the magnesium alloy may be a solid solution alloy or a precipitation hardening type alloy.

In the first aspect, the magnesium alloy may include at least one alloy element selected from Ca, Zn, Al, Sn, Mn, Si, Sr, Li, In, Ga, Ba, Ce, La, Nd, Gd or Y.

In the first aspect, the specific crystal plane may be (0001), (10-10), (2-1-10); a crystal plane which is crystallographically the same as the crystal planes; or a crystal plane with an atomic packing density of 0.4 or more.

According to the first aspect of the present invention, there is a method to control the biodegradation rate of magnesium, wherein the biodegradation rate is determined by controlling the atomic packing density of a surface in contact with a living body.

In the second aspect, the magnesium may include pure magnesium or a magnesium alloy.

In the second aspect, the magnesium may be polycrystalline magnesium, and may include a texture which is preferentially oriented in a specific crystal plane.

In the second aspect, the magnesium alloy may be a solid solution alloy or a magnesium precipitation hardening type alloy.

In the second aspect, the magnesium alloy may be comprised of at least one alloy element selected from Ca, Zn, Al, Sn, Mn, Si, Sr, Li, In, Ga, Ba, Ce, La, Nd or Gd.

In the second aspect, the magnesium may be single crystal magnesium, and the surface contacting the living body is (0001), (10-10), (2-1-10); a crystal plane which is crystallographically the same as the crystal planes; or a crystal plane with an atomic packing density of 0.4 or more.

In the second aspect, the specific crystal plane may be (0001), (10-10), (2-1-10): a crystal plane which is crystallographically the same as the crystal planes: or a crystal plane with an atomic packing density of 0.4 or more.

In the third aspect of the present invention, there is provided a biodegradable article made of the magnesium according to the first aspect, wherein a plane of the article contacting a living body is oriented with a crystal plane in which the degradation rate is relatively slow.

In the third aspect, the article may be a medical article.

In the third aspect, the article may be an implant or a stent.

### [Advantageous Effects]

According to one embodiment of the present invention, the degradation rate of magnesium can be controlled by processing magnesium single crystal so that a specific crystal plane is oriented toward a surface in contact with a living body by using the crystallographic anisotropy of the magnesium single crystal.

Also, according to another embodiment of the present invention, the degradation rate of magnesium can be controlled by forming a texture in the polycrystalline magnesium and controlling the crystallographic plane and/or the degree of the preferential orientation (i.e. degree of texturization).

### [Description of Figures]

FIG. 1 shows the state of a crystallographic plane according to the processing of magnesium according to an embodiment of the present invention.
FIG. 2 is a schematic view of rotation angles and the corresponding crystallographic planes in eleven single crystal magnesium specimens manufactured according to an embodiment of the present invention.
FIG. 3 is a graph showing the results of a potentiodynamic polarization test in eleven single crystal magnesium specimens manufactured according to an embodiment of the present invention.
FIG. 4 shows the pitting potential obtained from the polarization curve of FIG. 3.
FIG. 5 is a graph showing the results of a potentiostatic test in eleven single crystal magnesium specimens manufactured according to an embodiment of the present invention.
FIG. 6 shows comparison results of the relative corrosion amounts obtained through a potentiostatic test.
FIG. 7 shows an impedance spectrum in a Nyquist plot obtained from a 3.5% NaCl solution of a magnesium single crystal specimen after exposure to an open circuit potential.
FIG. 8a to 8d show XPS test results of eleven single crystal magnesium specimens manufactured according to an embodiment of the present invention.

### [Best Mode]

The constitution and operation of the embodiments of the present invention will be described below in more detail with reference to the accompanying drawings.

In the following descriptions of the present invention, if detailed descriptions of known functions or components are determined to make the subject matter of the present invention unnecessarily obscure, they will be omitted. Furthermore, when the present invention is described to be comprised of (or includes or has) some elements, it should be understood that it may be comprised of (or include or has) only those elements, or it may be comprised (or include or have) of those elements and other elements as well if there is no specific limitation.

The inventors of the present invention conducted studies to lower the in vivo corrosion rate of magnesium. As a result, it was found that the in vivo degradation rates of magnesium differed considerably according to the atomic packing density of a surface in contact with a living body; when the magnesium is processed by using crystallographic anisotropy of the in vivo degradation rate of magnesium; and using magnesium single crystal or polycrystalline magnesium with a texture preferentially oriented in a specific crystallographic direction.

In the case where a control is performed such that a crystallographic plane of a surface in contact with a living body becomes a specific crystallographic plane or mainly contacts the specific crystallographic plane, the in vivo degradation rate of magnesium may be controlled to decrease or increase to such a degree available industrially according to needs, leading to the present invention.

In the present invention, the term "magnesium" includes pure magnesium and magnesium alloys containing 20 wt. % or less of alloying elements.

The alloying element may be configured to include a component or content not harmful to the living body when alloyed with magnesium, and may preferably contain at least one of the following: Ca, Zn, Al, Sn, Mn, Si, Sr, Li, In, Ga, Ba, Ce, La, Nd, Gd or Y.

Also, when alloyed with alloying elements, the magnesium alloys are preferably solid solution alloys or precipitation hardening type alloys.

The crystallographic anisotropy of the magnesium may be obtained through single crystal magnesium, which may be produced by, for example, the Bridgman method. Specifically, when a medical device is processed with the single crystal material, by processing a surface in contact with a living body so as to have a specific crystal plane, the degradation rate of the surface of the processed medical device is different depending on the crystallographic plane.

In order to lower the degradation rate of the magnesium surface in contact with the living body in processing a medical device with a single crystal, the medical device may be processed such that the crystallographic plane becomes a plane with high atomic packing density, such as the (0001) plane, (10-10) plane, (2-1-10) plane or a plane which is crystallographically the same as these planes. To the contrary, to increase the degradation rate of a surface in contact with a living body, the medical device may be processed so that the crystallographic plane becomes a plane with low atomic packing density, such as the (51-60) plane and (21-30) plane.

The aforementioned subject matter is to be considered illustrative, not restrictive, and the appended claims are intended to cover all such modifications, enhancements, and other embodiments, which fall within the true spirit and scope of the present invention. Thus, to the maximum extent permitted by law, the scope of the present invention is to be determined by the broadest possible interpretation of the following claims and their equivalent, and shall not be restricted or limited by the previously mentioned detailed descriptions.

Also, the produced medical device may be processed such that a specific surface has a degradation rate faster or slower than other surfaces by variously adjusting the crystallographic orientation of the surface in contact with a living body.

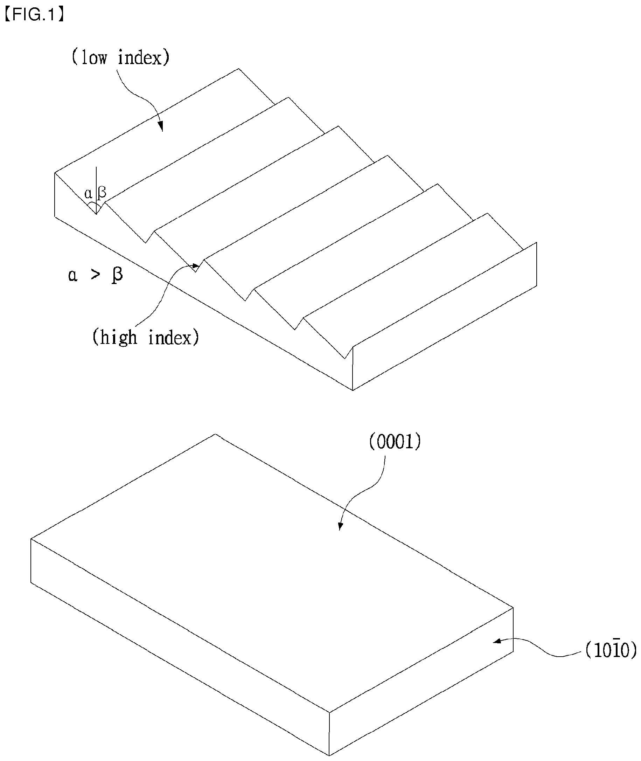

Furthermore, as shown in FIG. 1, by using a combination structure in which faces with a high surface index and surfaces with a low surface index are alternately repeated, the degradation rate can be finely controlled.

Moreover, the crystallographic anisotropy of magnesium can be obtained through polycrystalline magnesium with a texture, which is preferentially oriented in a specific crystallographic direction. Such magnesium may be produced through a plastic working process such as extrusion, rolling, and drawing.

Specifically, the medical device may be processed so that a texture preferentially oriented with a specific crystallographic plane is obtained and surfaces of the medical device in contact with a living body become specific crystallographic planes. In this case, the degradation rate of the surface of the processed medical device may differ depending on the type and/or degree of orientation (i.e. texturization) of the preferentially oriented crystallographic planes of the texture.

In order to lower the degradation rate of the surface in contact with the living body in processing a medical device with the single crystal, the medical device may be processed such that the crystallographic plane becomes a plane having a high atomic packing density, such as the (0001) plane, (10-10) plane, (2-1-10) plane or a plane which is crystallographically the same as these planes. To the contrary, to increase the degradation rate of a surface in contact with the living body, the medical device may be processed such that the crystallographic plane becomes a plane with a low atomic packing density of 0.2 or less--preferably 0.1 or lesssuch as the (51-60) plane and (21-30) plane.

The medical device which is processed such that a specific crystallographic plane of magnesium is formed on a surface thereof may be a medical screw, tube insert, medical bolt, medical plate, medical staple, medical tubular mesh, medical stent or medical coil.

Furthermore, the medical device which is processed such that a specific crystallographic plane of magnesium is formed on a surface thereof may be an implant. Examples of an implant include a spinal interbody spacer, bone filler, bone plate, bone pin, bone screw, scaffold and dental implant.

### [Mode for Invention]

The present inventors carried out the following experiments to confirm differences in corrosion characteristics according to a specific crystallographic plane of single crystal magnesium.

First, a magnesium single crystal was manufactured by so called "vertical Bridgman method" in which a pure magnesium (pure: purity of not less than 99.9 wt%; impurities: 0.006 wt. % of Al, 0.01 wt. % of Mn, 0.005 wt. % of Si, 0.004 wt. % of Fe and 0.003 wt. % of Cu) ingot was placed in a graphite mold of a conical shape or another specific shape, the mold was loaded in a vertical resistance furnace. The graphite mold in which the ingot was placed in was put in a cylindrical glass container in an environment blocked from ambient air, heated to a temperature higher than the melting point, and descended in the furnace. A nucleus was created from the sharp end or specific end of a cone or a specific shape in a specific direction, grown throughout the entire crystal, then grown as a single crystal, wherein the descending speed was set to 5 mm/h or less.

The crystallographic orientation of the manufactured magnesium single crystal was measured using the Laue back-reflection method. The single crystal was cut using a spark erosion machine to obtain 11 specimens with various crystallographic orientations.

The manufactured eleven specimens were mechanically polished using SiC abrasive paper and annealed in a vacuum pyrex tube to remove dislocations generated during the machining process. Finally, oxides formed on the surfaces of the manufactured specimens were chemically removed by using a mixture solution of CH₃OH and HNO₃ mixed at a volume ratio of 2:1.

In order to confirm the influence of the crystallographic orientation on the corrosion behavior, 11 specimens with a plane perpendicular to the direction from the [0001] direction to the [10-10] or [2-1-10] direction were prepared.

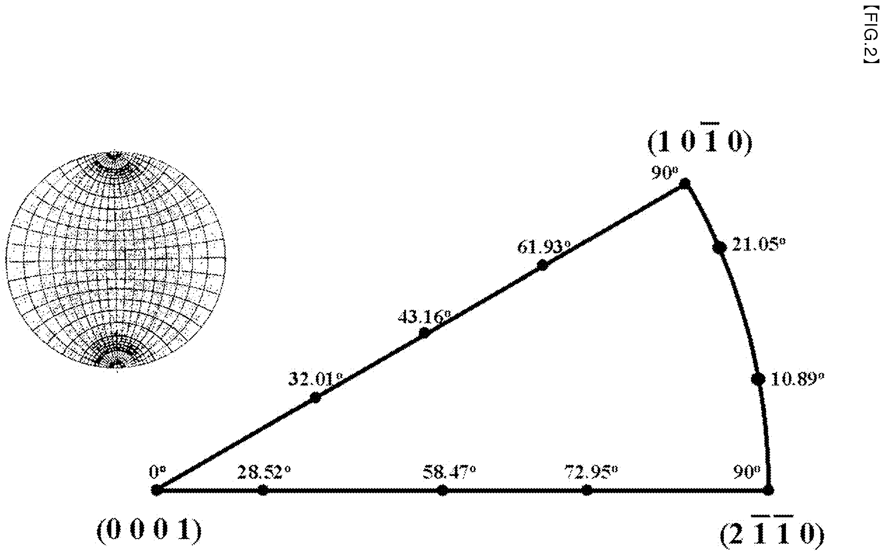

FIG. 2 is a schematic view of the crystallographic orientation of the specimens. Details of the index of the surface corresponding to each rotation angle are shown in Table 1 below.

**[Table 1]**

| No. | Miller index of cut plane | Miller index of cut plane | Rotation Angle (º) | Atomic Packing Density (atom/Å²) |
|---|---|---|---|---|
| | (3 index) | (4 index) | | |
| 1 | (001) | (0001) | 0.00 | 0.9069 |
| 2 | (103) | (10-13) | 32.01 | 0.2561 |
| 3 | (102) | (10-12) | 43.16 | 0.3302 |
| 4 | (101) | (10-11) | 61.93 | 0.4255 |
| 5 | (100) | (10-10) | 90.00 | 0.4818 |
| 6 | (510) | (51-60) | 21.05 | 0.0865 |
| 7 | (210) | (21-30) | 10.89 | 0.1821 |
| 8 | (110) | (11-20) | 90.00 | 0.5563 |
| 9 | (111) | (11-21) | 72.95 | 0.2659 |
| 10 | (112) | (11-22) | 58.47 | 0.4742 |
| 11 | (116) | (11-26) | 28.52 | 0.2656 |

Eleven prepared specimens were immersed in a 3.5% NaCl aqueous solution to perform an electrochemical test. The electrochemical tests were carried out using an electrochemical evaluation consisting of a potentiodynamic polarization test and a potentiostatic test. At this time, a saturated calomel electrode and two pure graphite rods were used as a reference electrode and a counter electrode.

For the direct current measurement, the potentiodynamic polarization test was performed using an EG&G PAR 263A potentiostat. The specimens were immersed in a solution for one hour to stabilize the open circuit potential prior to conducting the potentiodynamic polarization test.

The potential of the electrode was changed at a rate of 0.166 mV/s in a range from the initial potential of 250 mV to the final pitting potential. The surface layers of the specimens subjected to the corrosion test were maintained at the open circuit voltage for one hour and analyzed using XPS.

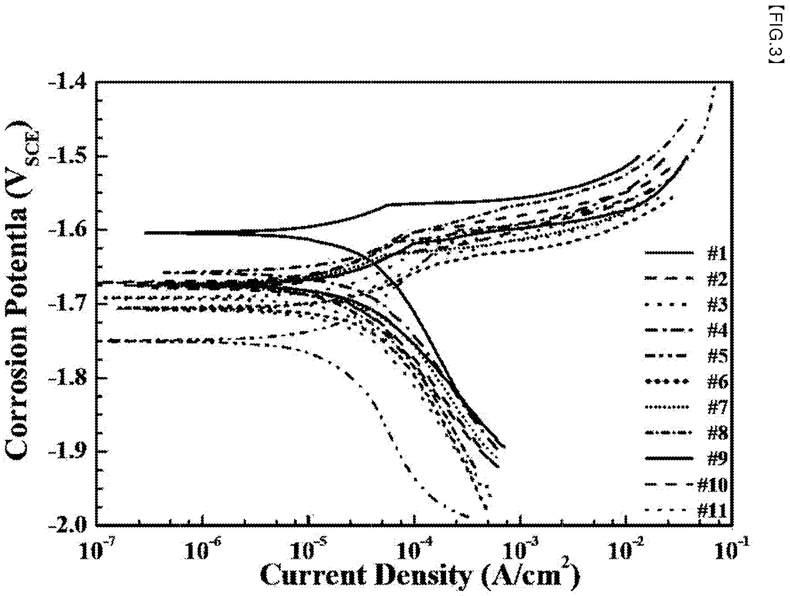

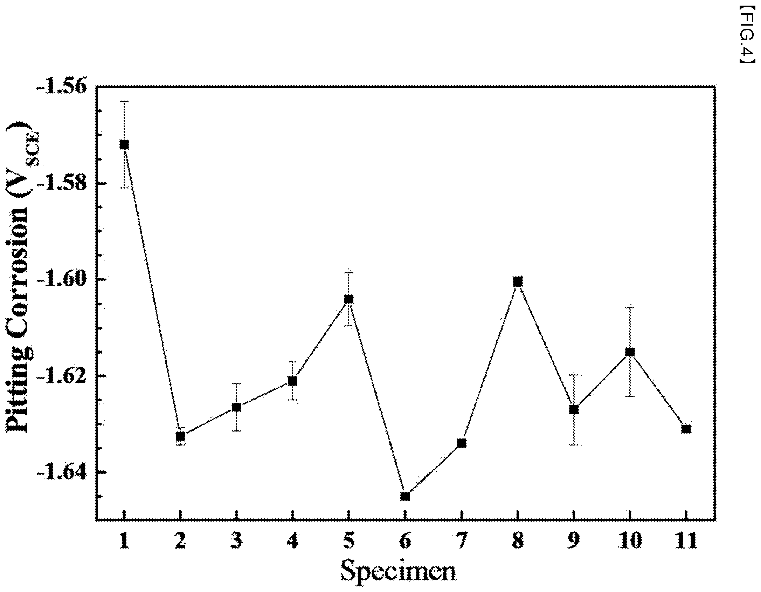

FIG. 3 shows the potentiodynamic polarization curves of magnesium single crystals with various crystallographic orientations. FIG. 4 shows pitting potentials obtained from the polarization curve of FIG. 3. As seen in FIG. 3, pitting occurred in all crystallographic orientation specimens immersed in a 3.5% NaCl aqueous solution.

As a result of the potentiodynamic polarization test, the pitting potential tends to decrease from -1.57 V_{SCE} to -1.63 V_{SCE} as the rotation angle increases to 32° from the first (0001) plane, while the pitting potential tends to increase to -1.60 V_{SCE} as the rotation angle increases to 90º in the (10-10) plane. It can be seen that such a change in the pitting potential shows a tendency that almost coincides with the change in the atomic packing density shown in Table 1 above. This change in the pitting potential implies that when controlling the crystallographic orientation of the magnesium surface, the pitting resistance of the magnesium surface can be controlled at various values.

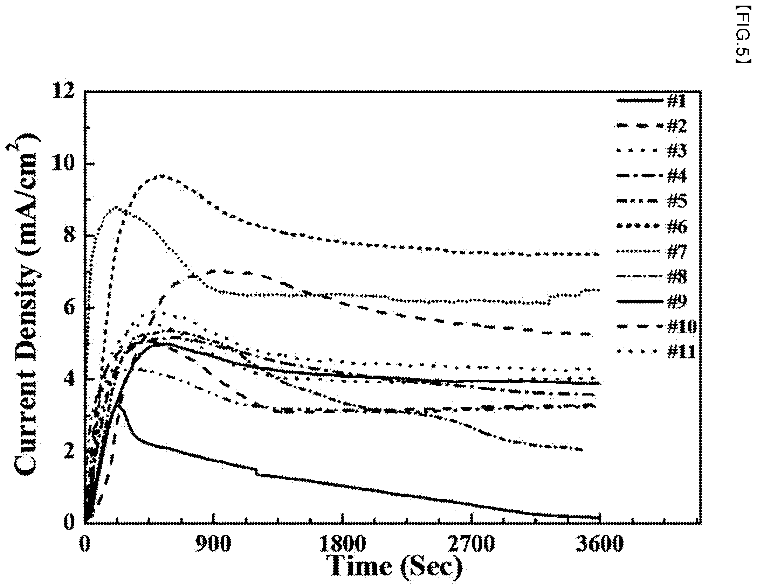

Next, a potentiostatic measurement was performed to further verify the effect of the crystallographic orientation on the corrosion characteristics. FIG. 5 shows results of the potentiostatic test performed at a potential of the -1.57 V_{SCE}.

In the potentiostatic test according to an embodiment of the present invention, the potential applied to the pitting potential of the (0001) plane, and a change in the current density according to time in FIG. 5 was related to the start and propagation of the pitting.

As can be seen in FIG. 5, in the potentiostatic test, the pitting resistance is highest in the (0001) plane. The resistance decreases as the rotation angle increases from 0° to 32°, and the resistance increases as the rotation angle increases to 90°.

As such, the result in which the amount of corrosion increases as the rotation angle increases, and the amount of corrosion decreases as the rotation angle further increases to 90° shows the same tendency as the results of the potentiodynamic polarization test. Such a result is determined due to generation of a difference in the stability of a film formed on a surface of each specimen, and thus, differences in corrosion resistance performance are generated.

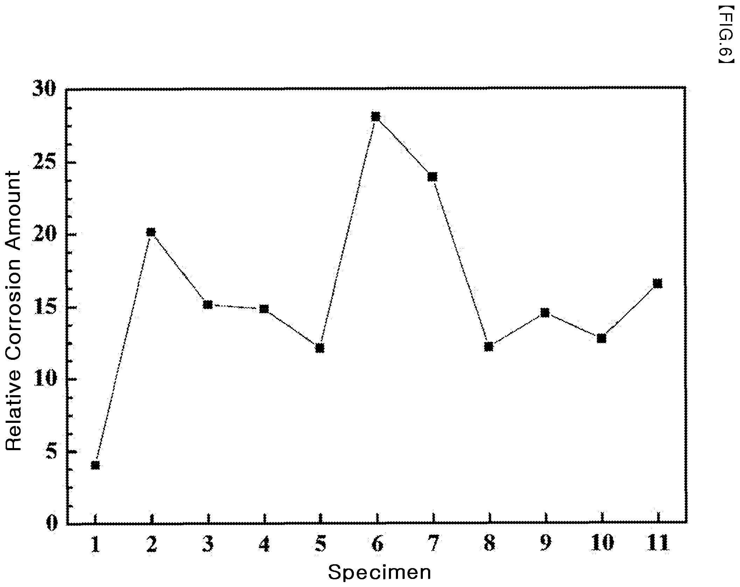

FIG. 6 shows results of the measurement of the amount of corrosion in specimens which were corroded by the potentiostatic test (the amount of corrosion when it is assumed that the fully corroded state is 100). As shown in FIG. 6, the corrosion amount of specimen 1 is the smallest, with the amounts of the corrosion of specimens 5, 8 and 10 similarly small, and the amounts of corrosion of specimens 2, 6 and 7 are large.

These results indicate that the films generated at 0°, 90°, or surfaces adjacent thereto tend to effectively block the invasion of chlorine ions.

As can be also seen from FIG. 6, there is a difference of about 7 times between the maximum value and the minimum value in terms of the corrosion amount. Such a difference indicates that the in vivo corrosion rate can be controlled to a considerable degree by controlling the crystallographic direction of the crystal plane of the single crystal.

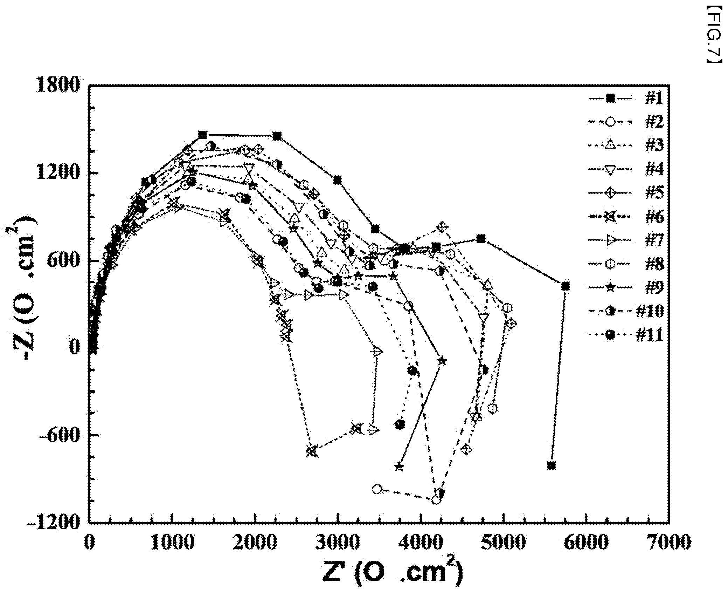

FIG. 7 shows an impedance spectrum in a Nyquist plot obtained from magnesium single crystal specimens in a 3.5% NaCl solution after exposure to an open circuit potential. In FIG. 7, recessed portions in the semicircular shapes are due to the non-uniformity and roughness of surfaces, and the pitting corrosion of specimens 1-11. The arc of the specimen with a rotation angle of 0° or 90° is larger in diameter than the other specimens, which means that the pitting resistance of the specimen is larger than the other specimens. That is, it means that the 0° or 90° specimen has the formation of a passive film more accelerated than the other specimens.

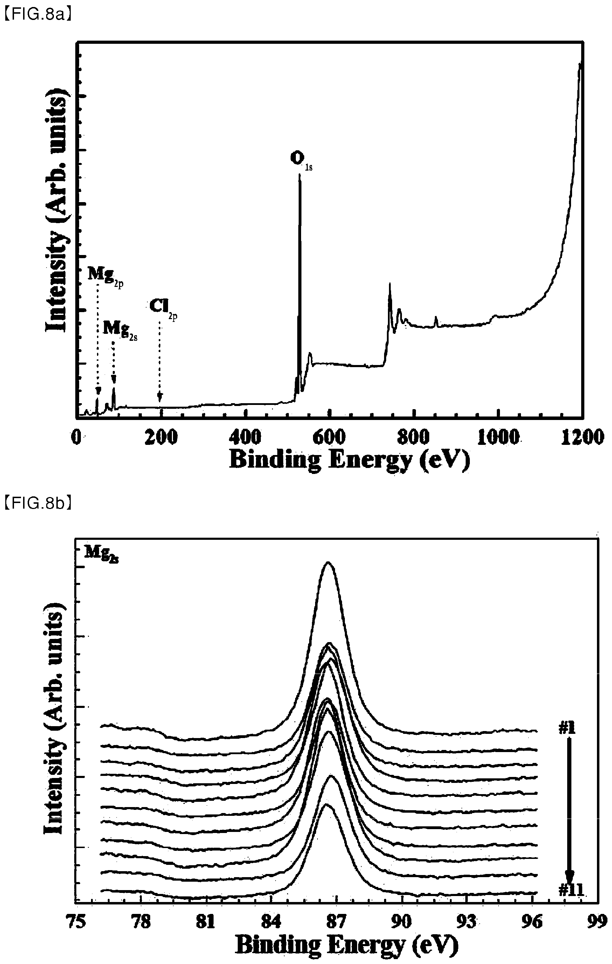

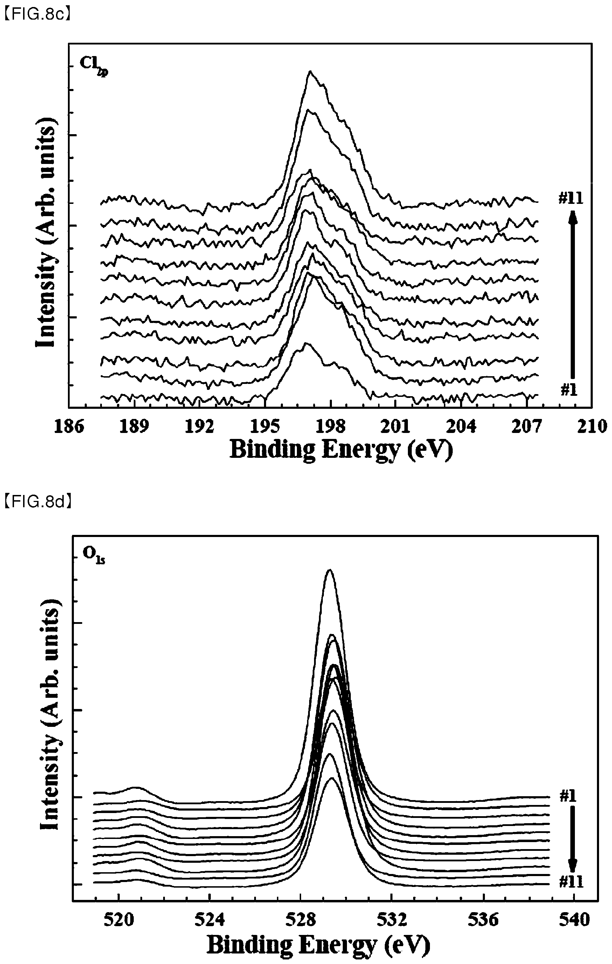

The chemical compositions of the surface films were analyzed by XPS after one hour of exposure at the open circuit potential. FIG. 8 shows XPS analysis results of the specimens according to an embodiment of the present invention.

FIG. 8a shows an XPS result of the 0° specimen. In the surface film, peaks corresponding to Mg, Cl and O were detected. The Mg₂ₛ peak as shown in FIG. 8b indicates that magnesium of the surface film exists in the form of MgO and Mg(OH)₂. Also, FIG. 8d shows that the intensity of the O₁ₛ peak decreases as the angle of the specimen increases from 0° to 32°, and then increases as the angle of the specimen increases to 90°. The intensity of the Mg peak was small in the 32° specimen, and the intensity of Cl peak was the highest in the 32° specimen.

As a result of the XPS analysis, the passivation film formed on the surface of magnesium is MgO/Mg(OH)₂.

Meanwhile, when the magnesium specimens were immersed in the NaCl 3.5% solution, chloride ions penetrated into the hydroxide film and the magnesium was corroded. As a result, MgCl₂ was produced and magnesium was dissolved through pores existing in a corrosion product MgCl₂, and diffused to the outside. This corrosion mechanism means that the degree of bonding in the magnesium oxide (MgO and Mg(OH)₂) formed on the surface of the magnesium may have a very large influence on the corrosion resistance. In other words, as the stability of the passivation film increases, the adsorption of chlorine ions can be prevented and the corrosion resistance can be improved.

It can be seen that the results of the potentiodynamic polarization test mentioned above and the potentiostatic test have a close correlation with the atomic packing density of the cut surface in Table 1 above. That is, specimens 1, 5, 8, and 10, which have high atomic packing densities, have a relatively low corrosion rate, while specimens 2, 6, and 7, which have low atomic packing densities, have a relatively fast corrosion rate.

This is confirmed by the intensity of the Cl peak of the XPS spectrum in single crystal specimens with different crystallographic orientations. In the specimens with low atomic packing densities, the intensity of the Cl peak is high, which means that the chlorine ions easily penetrated.

From the results above, it can be seen that in the case of a surface processed with a low atomic packing density (high Miller index), the spacing between the atoms is so large that it is difficult to effectively block a metal-metal bonding or a corrosion product formed on a surface. In contrast, in the case of a surface processed with a high atomic packing density (low Miller index), such as the Oº or 90° specimen, the spacing between the atoms is narrow, and thus, a passive film with a higher corrosion resistance is formed.

### [INDUSTRIAL APPLICABILITY]

The present invention can be applied to the medical field requiring biodegradability such as an implant or a stent.

## Claims

1. Biodegradable magnesium having a biodegradation rate which is determined by controlling an atom packing density of a surface contacting a living body.

2. The biodegradable magnesium according to claim 1,
wherein the magnesium is pure magnesium or a magnesium alloy.

3. The biodegradable magnesium according to Claim 1 or 2,
wherein the magnesium is polycrystalline magnesium, and the magnesium comprises a texture which is preferentially oriented with a specific crystallographic plane.

4. The biodegradable magnesium according to Claim 2,
wherein the magnesium alloy is a solid solution alloy or a precipitation hardening type alloy.

5. The biodegradable magnesium according to Claim 2,
wherein the magnesium alloy comprises at least one selected from Ca, Zn, Al, Sn, Mn, Si, Sr, Li, In, Ga, Ba, Ce, La, Nd, Gd or Y.

6. The biodegradable magnesium according to Claim 1 or 2,
wherein the magnesium is a single crystal magnesium, and the surface in contact with the living body corresponds to (0001), (10-10), (2-1-10); or a crystal plane which is crystallographically the same as the crystal planes; or a crystal plane with an atomic packing density of 0.4 or more.

7. The biodegradable magnesium according to Claim 3,
wherein the specific crystal plane is (0001), (10-10), (2-1-10); a crystal plane which is crystallographically the same as the crystal planes; or a crystal plane with an atomic packing density of 0.4 or more.

8. A method for controlling the biodegradation rate of magnesium,
wherein the biodegradation rate is determined by controlling an atomic packing density of the surface in contact with a living body.

9. The method according to Claim 8,
wherein the magnesium is pure magnesium or a magnesium alloy.

10. The method according to Claim 8 or 9,
wherein the magnesium is polycrystalline magnesium, and the magnesium is preferentially oriented in the specific crystallographic plane.

11. The method according to Claim 9,
wherein the magnesium alloy is a solid solution alloy or a magnesium precipitation hardening type alloy.

12. The method according to Claim 9,
wherein the magnesium alloy comprises at least one selected from Ca, Zn, Al, Sn, Mn, Si, Sr, Li, In, Ga, Ba, Ce, La, Nd or Gd.

13. The method according to Claim 8 or 9,
wherein the magnesium is a single crystal magnesium, and the surface in contact with the living body is (0001), (10-10), (2-1-10); a crystal plane which is crystallographically the same as the crystal planes; or a crystal plane with an atomic packing density of 0.4 or more.

14. The method according to Claim 10,
wherein the specific crystal plane is (0001), (10-10), (2-1-10); a crystal plane which is crystallographically the same as the crystal planes; or a crystal plane with an atomic packing density of 0.4 or more.

15. A biodegradable article made of the magnesium set forth in any of Claims 1 to 7,
wherein the surface of the article in contact with a living body is oriented with a crystallographic plane in which the degradation rate is relatively slow by controlling the atomic packing density of the specific crystal plane.

16. The biodegradable article according to Claim 15,
wherein is an article for medical use.

17. The biodegradable article according to Claim 16,
wherein the article is an implant or a stent.
